# EUROPEAN PATENT SPECIFICATION

(11) **EP 0 384 463 B1**
(45) Date of publication and mention of the grant of the patent: **10.08.1994**
(21) Application number: 90103454.6
(22) Date of filing: 22.02.1990
(51) Int. Cl.: C07C 265/14, C07C 263/10

(54) **Preparation process of xylylene diisocyanate**
Verfahren zur Herstellung von Xylylendiisocyanat
Procédé pour la préparation du xylylènediisocyanate

(30) Priority: 23.02.1989 JP 41495/89; 01.03.1989 JP 46410/89
(43) Date of publication of application: 29.08.1990
(73) Proprietor: MITSUI TOATSU CHEMICALS, Inc., Chiyoda-Ku Tokyo 100 (JP)
(72) Inventor: Nagata, Teruyuki, Omuta-Shi, Fukuoka-Ken, 836 (JP); Wada, Masaru, Omuta-Shi, Fukuoka-Ken, 837 (JP); Mizuta, Hideki, Omuta-Shi, Fukuoka-Ken, 837 (JP)
(74) Representative: VOSSIUS & PARTNER

(56) References cited:
- DE-A- 1 950 784
- FR-A- 1 468 510
- GB-A- 1 162 155

## Description

The present invention relates to a process for the preparation of xylylene diisocyanate by reacting xylylene diamine hydrochloride with phosgene in the presence of an ester.

Xylylene diisocyanate is a very useful compound as a raw material for polyurethane-based materials, polyurea-based materials and polyisocyanurate-based materials in chemical, resin and paint industries.

Various methods have already been proposed for the preparation of xylylene diisocyanate.

Processes to prepare isocyanates by reacting organic amines with phosgene, that is, so-called phosgenation processes, have been proposed. Processes which have also been proposed other than phosgenation processes are, for example, a process for reacting aromatic compounds containing chloromethyl groups with an alkali cyanate in the presence of a copper catalyst
(JP-A-52-46042(1977)), and a process for the vapor phase oxidation of N-substituted formamides by an oxygen containing gas in the presence of a catalytic amount of metal
(JP-A-54-39018(1979), which corresponds to US-A-4207251 and EP-A-602).

Additionally, phosgenation processes which prepare isocyanates by conducting the reaction of organic primary amines with phosgene in an inert solvent have been known.

In these processes, aromatic primary amines can be converted with comparative ease to high-purity aromatic isocyanates by passing phosgene gas through a suspension of the free aromatic amines, their carbonates or hydrochlorides in the solvent (e.g. see GB-A-1 037 933).

On the other hand, aliphatic primary amines generally require lengthy times in the reaction with phosgene as compared to aromatic primary amines, and also generate, as well known in the art, chloroderivatives as by-products due to a deamination reaction.

Xylylene diamine is classified in the aliphatic diamine compound. In the preparation of xylylene diisocyanate by reacting xylylene diamine with phosgene according to a conventional process, a monoisocyanate is formed as a by-product as in the preparation of ordinary aliphatic isocyanates. The monoisocyanate is a chloroderivative (hereinafter referred to as chlorinated impurity) having the formula (I): 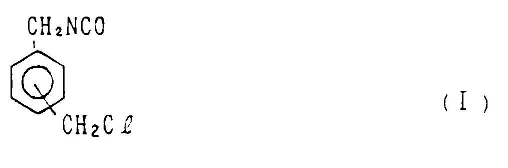
The chlorinated impurity is usually formed in an amount of 3 to 10 % by weight and sometimes goes up to 20 % by weight. Hence the yield of the desired product undergoes a corresponding decrease.

When xylylene diisocyanate contains the chlorinated impurity, the chlorinated impurity affects the reaction of isocyanate groups with active hydrogen containing compounds in the preparation or polyurethane resins from xylylene diisocyanate. The chlorinated impurity inhibits the reaction, accelerates gelation of the prepolymer and further exerts an adverse effect upon the properties of the resulting polyurethane resin.

No difference is generally observed between the properties of the chlorinated impurity and corresponding isocyanate except that the chlorinated impurity has a boiling point from 5 to 20°C lower than that of the isocyanate. Specific procedures are hence required for the separation and purification of xylylene diisocyanate as proposed in Japanese Patent Publication SHO 49-13786(1974).

The formation of the above chlorinated derivative is mostly not found in the preparation of aromatic isocyanates, and is observed in the preparation of aliphatic isocyanates.

Consequently, processes have been proposed in order to inhibit the formation of the impurity as much as possible and to enhance the efficiency of separation and purification steps. These processes include, for example, (1) a process for preparing isocyanates by reacting xylylene diamine with phosgene at 120 to 160 °C under pressure of 0.5 to 10 kg/cm²G (Japanese Patent Publication SHO 42-179797(1967), which corresponds to US-A-340227 and GB-A-1162155) , and (2) a process for converting xylylene diamine or its hydrochloride to xylylene diisocyanate at a reaction temperature of 120 to 160°C in a range of from 18/1 to 30/1 by weight ratio of solvent/amine raw material (GB-A-1036782). In the Examples of this application hydrocarbons and halogenated hydrocarbons are used as solvents. Esters are only mentioned inter alia,

These processes, however, have been disadvantageous in that extensive equipment is required for the preparation because the reaction is conducted under pressure, and uses a large amount of solvent which leads to low volume efficiency and very poor economy. As a result, conventional processes have been unsatisfactory in view of industrial production.

FR-A-1 468 510 describes that xylylene diisocyanate can be obtained by adding a specific surface active agent to the suspensions of primary amine hydrochlorides in liquid organic diluents which subsequently are phosgenated. Butyl acetate is added to make the mixture more fluid.

The object of this invention is to provide a process for preparing xylylene diisocyanate by reacting xylylene diamine hydrochloride with phosgene that yields good efficiency in industrial scale whereby the formation of the by-product, that is, the chlorinated impurity having the above formula (I) is inhibited.

This object is achieved on the basis of the surprising finding that xylylene diisocyanate having a very small content of the chlorinated impurity can be prepared by using an ester as a reaction solvent.

That is, the subject matter of this invention is a process for the preparation of xylylene diisocyanate by reacting xylylene diamine hydrochloride with phosgene in the presence or an ester as a reaction solvent characterized in that xylylene diamine is reacted with hydrogen chloride gas in a first step at a temperature of 30°C or less, and in a second step the resultant xylylene diamine hydrochloride is reacted with phosgene at a temperature of from 120 to 170°C.

Xylylene diamine used in the preparation of its hydrochloride is m-xylylene diamine, p-xylylene diamine, o-xylylene diamine or a mixture containing various proportions of these isomers. Accordingly, unless otherwise noted hereinafter, the term "xylylene diamine" includes any of these amines and the isocyanates prepared from these amines are referred to as xylylene diisocyanate.

In the process of this invention, xylylene diamine is used as the raw-material in the form of the hydrochloride.

The process of this invention has a remarkable characteristic of reacting xylylene diamine hydrochloride with phosgene in the presence of an ester is a reaction solvent.

Consequently, xylylene diamine hydrochloride can be used as a raw material for the reaction with phosgene. Alternately, free xylylene diamine can be used as the raw material, converted to hydrochloride in the reaction system and then can be subjected to reaction with phosgene.

The reaction solvent used in the process of this invention is an ester. Various kinds of esters can be used, carboxylic acid esters and fatty acid esters and aromatic carboxylic acid esters are preferred in particular. Exemplary fatty acid esters include amyl formate, n-butyl acetate, isobutyl acetate, n-amyl acetate, isoamyl acetate, methylisoamyl acetate, methoxybutyl acetate, sec-hexyl acetate, 2-ethylbutyl acetate, 2-ethylhexyl acetate, cyclohexyl acetate, methylcyclohexyl acetate, benzyl acetate, ethyl propionate, n-butyl propionate, isoamyl propionate, ethyl acetate, butyl stearate, butyl lactate and amyl lactate. Aromatic carboxylic acid esters include, for example, methyl salicylate, dimethyl phthalate and methyl benzoate. More preferred esters are aliphatic esters having a boiling point of 120 to 170 °C under atmospheric pressure. The use of these esters is preferred in view of preventing the decomposition of isocyanates due to overheating. These solvents can be used singly or in combination.

The amount of the solvent used in the process of this invention is preferably in a weight ratio of solvent/raw material amine in the range of 8/1 to 16/1.

When the weight ratio is less than 8/1, a large amount of amine hydrochloride is deposited and the reaction mixture becomes difficult to stir. On the other hand, a weight ratio exceeding 16/1 has little effect on the acceleration of the reaction rate and requires larger amounts of the solvent. Hence thermal efficiency in the concentration step is reduced and volume efficiency becomes industrially unfavorable.

The reaction can be carried out by using free xylylene diamine as a raw material, reacting it with hydrogen chloride gas in an ester type solvent at a temperature of 30 °C or less to form the hydrochloride and then reacting the hydrochloride with phosgene at a temperature of from 120 to 170 °C, or by using xylylene diamine hydrochloride as a raw material and reacting it with phosgene in an ester type solvent at a temperature of from 120 to 170 °C.

In the above reaction method, xylylene diamine is first reacted with hydrogen chloride gas in an ester type solvent to form xylylene diamine hydrochloride. The temperature during the formation of xylylene diamine hydrochloride is 30°C or less. When the temperature exceeds 30 °C, the chlorinated impurity is liable to increase. When the temperature is 30 °C or less, good results can be obtained even in the vicinity of 0 °C. Although hydrochloride formation can also be conducted at lower temperatures than this range, it is unfavorable because extensive refrigeration is required. The reason for the good results obtained by conducting hydrochloride formation at 30 °C or less is not clear, but is thought that the results depend upon the solubility and particle size of xylylene diamine hydrochloride.

The temperature for reacting the resultant xylylene diamine hydrochloride with phosgene is from 120 to 170 °C, preferably from 130 to 150 °C.

Further, in the other above mentioned reaction method, xylylene diamine hydrochloride is used as a raw material and is reacted with phosgene in an ester type solvent.

The reaction temperature is the same as above, that is, from 120 to 170 °C, preferably from 130 to 150 °C.

When phosgenation is conducted for a long time at a temperature exceeding 170°C in any of the above methods, decomposition of the resultant xylylene diisocyanate results from poor thermal stability and leads to an increase in tar content and a decrease in yield. When the reaction temperature rises, formation of the chlorinated impurity, that is, chloromethylbenzyl isocyanate (hereinafter abbreviated as CBi), tends to increase. A reaction temperature lower than 120 °C is unfavorable because the reaction rate becomes too slow.

In the process of this invention, the reaction is usually carried out under atmospheric pressure. In order to increase the reaction rate and to inhibit the formation of CBi, the isocyanate can also be prepared under increased pressure.

Typical preferred embodiments of the process of this invention will be illustrated hereinafter.

In the practical procedures corresponding to the above reaction method, for example, a reaction vessel equipped with a reflux condenser, thermometer, phosgene inlet tube and a stirrer is charged with xylylene diamine as a raw material and an ester as a reaction solvent.

For example, xylylene diamine as the raw material and an ester as a reaction solvent are charged to the reaction vessel, and a prescribed amount of hydrogen chloride gas is blown into the suspension of xylylene diamine while controlling the internal temperature at 30°C or less to form xylylene diamine hydrochloride. Thereafter the reaction mixture is heated to a prescribed temperature and phosgene is introduced to complete the conversion to isocyanate.

In the procedures corresponding to the other above mentioned reaction method, for example xylylene diamine hydrochloride as a raw material and an ester as a reaction solvent are charged to a reaction vessel and phosgene is introduced to the reaction mixture to complete the conversion to isocyanate at a prescribed temperature.

After completing the reaction in each of the above methods, unreacted phosgene and hydrogen chloride are purged with nitrogen and the solvent is removed. The residue is distilled to obtain pure xylylene diisocyanate.

According to the process of this invention, xylylene diisocyanate having an extremely low content of CBi can be obtained. Accordingly, post treatment steps such as distillation purification can be simplified and the loss of product due to thermal deterioration in the post treatment step can be reduced. Consequently, the process of the present invention is industrially very valuable.

The present invention will hereinafter be illustrated in detail by way of examples and comparative examples.

### Comparative Example 1

To a 2ℓ reaction flask equipped with a reflux condenser, thermometer, phosgene or hydrogen chloride inlet tube, and a stirrer, 136.2 g (1.0 mole) of m-xylylene diamine raw material (hereinafter abbreviated as m-XDA) and 1200 g of amyl acetate as a solvent were charged.

Then 80 g of hydrogen chloride gas was blown into the flask over 2 hours with stirring and cooling. The internal temperature rose to 60 °C. The resulting mixture was successively heated to 135 °C and phosgene was introduced at a rate of 25 g/hr. The reaction was continued for 15 hours while maintaining the temperature from 135 to 140 °C.

After completing the reaction, unreacted phosgene and hydrogen chloride were purged with nitrogen. After removing the solvent, the residue was vacuum distilled at 133 to 266 Pa (1 to 2 mm Hg).

m-Xylylene diisocyanate (hereinafter abbreviated as m-XDi) containing 0.8 % by weight of m-chloromethylbenzyl isocyanate (hereinafter abbreviated as m-CBi) was obtained at a yield of 172.7 g. The yield converted to a purity basis was 91.0 %. The amount of CBi formed was 0.76 mol % per mole of m-XDA.

### Comparative Example 2

To the same reaction flask as used in Comparative Example 1, m-XDA and amyl acetate were charged by the same procedures as in Comparative Example 1 and cooled to 5 °C.

Phosgene was introduced at a rate of 200 g/hr for 3 hours while maintaining the internal temperature from 0 to 5 °C. Phosgene was further introduced at a rate of 20 g/hr and the reaction mixture was heated to 135 °C. Thereafter the reaction was conducted for 12 hours while maintaining the internal temperature from 135 to 140 °C.

After completing the reaction, the post-treatment was carried out by the same procedures as conducted in Comparative Example 1. m-XDi obtained was 170.7 9. The yield converted to a purity basis was 89.6 %. The m-XDi contained 1.2 % m-CBi by weight. The amount of CBi formed was 1.13 mol % per mole of m-XDA.

### Comparative Examples 3-6

The reaction and post-treatment were carried out by the same procedures as conducted in Comparative Example 1 except that the solvents illustrated in Table 1 were used. The effect of the solvents on the amount of m-CBi formed was investigated. Results are illustrated in Table 1.

**Table 1**

| | Solvent | m-XDi yield (mol %/m-XDA) | m-CBi amount (mol %/m-XDA) |
|---|---|---|---|
| Comparative Example 3 | Hexyl acetate | 90.8 | 0.78 |
| Comparative Example 4 | o-Dichlorobenzene | 87.7 | 3.55 |
| Comparative Example 5 | Mesitylene | 86.2 | 3.80 |
| Comparative Example 6 | DMi | 50.8 | 15.4 |
| Note: DMi is 1,3-dimethyl-2-imidazolidinone | | | |

### Example 1

To a 2 ℓ reaction flask equipped with a reflux condenser, thermometer, phosgene or hydrogen chloride inlet tube and a stirrer, 136.2 g (1.0 mole) of m-XDA raw material and 1200 g of amyl acetate as a solvent were charged.

Then 80 g of hydrogen chloride gas was blown into the flask over 2 hours with stirring and cooling while maintaining the internal temperature from 25 to 30 °C to form hydrochloride of m-XDA. Then the reaction mixture was heated to 135 °C.

After raising the temperature, phosgene was introduced to the reaction mixture at a rate of 25 g/hr and the reaction was continued for 15 hours while maintaining the temperature from 135 to 140 °C.

After completing the reaction, unreacted phosgene and hydrogen chloride were purged with nitrogen, and the solvent was removed. The residue was vacuum distillated at 133 to 266 Pa (1 to 2 mm Hg). m-XDi containing 0.4 % m-CBi by weight was obtained in a yield of 173.3 g. The yield converted to a purity basis was 91.7 %. The amount of m-CBi formed was 0.38 mol % per mole of m-XDA.

### Examples 2-4 and Comparative Examples 7-8

The reaction and post-treatment were carried out by the same procedures as conducted in Comparative Example 1 except that the solvents and hydrochloride forming temperatures were used as illustrated in Table 2.
Results are illustrated in Table 2

**Table 2**

| | Solvent | Hydrochloride forming temperature (°C) | m-XDi yield (mol %/m-XDA) | m-CBi amount (mol %/m-XDA) |
|---|---|---|---|---|
| Example 2 | Amyl acetate | 0 - 5 | 91.2 | 0.40 |
| Example 3 | Hexyl acetate | 25 - 30 | 90.8 | 0.42 |
| Example 4 | Butyl propionate | 25 - 30 | 91.0 | 0.39 |
| Comparative Example 7 | o-Dichlorobenzene | 50 - 55 | 87.9 | 3.50 |
| Comparative Example 8 | o-Dichlorobenzene | 25 - 30 | 88.1 | 3.48 |

## Claims

1. A process for the preparation of xylylene diisocyanate by reacting xylylene diamine hydrochloride with phosgene in the presence of an ester as a reaction solvent characterized in that xylylene diamine is reacted with hydrogen chloride gas in a first step at a temperature of 30°C or less, and in a second step the resultant xylylene diamine hydrochloride is reacted with phosgene at a temperature of from 120 to 170°C.

2. The process according to claim 1, wherein the ester is an aliphatic ester.

3. The process according to claim 1, wherein the solvent is used in an 8/1 to 16/1 weight ratio of solvent to xylylene diamine hydrochloride.

## Patentansprüche

1. Verfahren zur Herstellung von Xylylendiisocyanat durch Umsetzung von Xylylendiaminhydrochlorid mit Phosgen in Gegenwart eines Esters als Reaktionslösungsmittel, gekennzeichnet dadurch, daß Xylylendiamin mit Chlorwasserstoffgas im ersten Schritt bei einer Temperatur von 30°C oder weniger umgesetzt wird, und im zweiten Schritt das entstandene Xylylendiaminhydrochlorid mit Phosgen bei einer Temperatur von 120 bis 170°C umgesetzt wird.

2. Verfahren nach Anspruch 1, bei dem der Ester ein aliphatischer Ester ist.

3. Verfahren nach Anspruch 1, bei dem das Lösungsmittel in einem Gewichtsverhältnis von 8/1 bis 16/1 des Lösungsmittels zu Xylylendiaminhydrochlorid verwendet wird.

## Revendications

1. Procédé de préparation de xylylènediisocyanate par réaction du chlorhydrate de xylylènediamine avec du phosgène, en présence d'un ester comme solvant réactionnel, caractérisé en ce qu'on fait réagir la xylylènediamine avec du chlorure d'hydrogène gazeux, au cours d'une première étape, à une température égale ou inférieure a 30°C, et, dans une seconde étape, on fait réagir avec du phosgène le chlorhydrate de xylylènediamine résultant, à une température de 120 à 170°C.

2. Procédé selon la revendication 1, dans lequel l'ester est un ester aliphatique.

3. Procédé selon la revendication 1, dans lequel le solvant est mis en oeuvre dans un rapport pondéral solvant/chlorhydrate de xylylènediamine qui vaut de 8/1 à 16/1.
